# FASCICULE DE BREVET EUROPEEN

(11) **EP 0 586 660 B1**
(45) Date de publication et mention de la délivrance du brevet: **29.01.1997**
(21) Numéro de dépôt: 93906660.1
(22) Date de dépôt: 24.03.1993
(51) Int. Cl.: A61K 7/13, C07C 323/36, C07C 323/41

(54) **METAPHENYLENEDIAMINES SOUFREES EN POSITION 2 POUR LA TEINTURE DES CHEVEUX**
METAPHENYLENDIAMINE MIT SCHWEFEL IN POSITION 2 ZUR HAARFÄRBUNG
2-SULPHURED METAPHENYLENEDIAMINES FOR DYEING HAIR

(30) Priorité: 25.03.1992 FR 9203614
(43) Date de publication de la demande: 16.03.1994
(73) Titulaire: L'OREAL, 75008 Paris (FR)
(72) Inventeur: JUNINO, Alex, F-93190 Livry-Gargan (FR); GENET, Alain, F-93600 Aulnay-sous-Bois (FR); LAGRANGE, Alain, F-78400 Chatou (FR)
(74) Mandataire: Casalonga, Axel
(86) Numéro de dépôt international: FR9300294
(87) Numéro de publication internationale: WO9318739

(56) Documents cités:
- DE-A- 3 343 642
- US-A- 4 085 217
- US-A- 4 146 688
- US-A- 4 260 634
- US-A- 4 973 760
- PATENT ABSTRACTS OF JAPAN vol. 004, no. 073 (C-012)28 Mai 1980

## Description

La présente invention est relative à l'utilisation pour la teinture de fibres kératiniques et plus particulièrement des cheveux humains, de métaphénylènediamines soufrées en position 2, à des compositions tinctoriales contenant ces métaphénylènediamines soufrées en position 2, à un procédé de teinture mettant en oeuvre ces compositions, ainsi qu'à de nouvelles métaphénylènediamines soufrées et leur procédé de préparation.

On a déjà utilisé des dérivés soufrés d'amines aromatiques, associés à des précurseurs de colorants d'oxydation, pour la teinture de fibres kératiniques.

Il est connu de teindre les fibres kératiniques et en particulier les cheveux humains, avec des compositions tinctoriales contenant des précurseurs de colorants d'oxydation et des coupleurs.

Les coupleurs, encore appelés modificateurs de coloration, permettent de faire varier les nuances obtenues avec les précurseurs de colorants d'oxydation.

Dans le domaine de la teinture des fibres kératiniques et en particulier des cheveux humains, on est à la recherche de coupleurs qui, associés à des précurseurs de colorants d'oxydation, permettent d'obtenir un large éventail de nuances, tout en conférant aux cheveux une coloration ayant une résistance satisfaisante à la lumière, au lavage, aux intempéries, à la transpiration et aux différents traitements que peuvent subir les cheveux.

La demanderesse vient de découvrir, ce qui fait l'objet de l'invention, que l'utilisation de certaines métaphénylènediamines soufrées en position 2, en tant que coupleurs avec des précurseurs de colorants d'oxydation de type ortho et/ou para dans des compositions tinctoriales pour fibres kératiniques, permettaient d'obtenir après application sur les fibres kératiniques et en particulier les cheveux humains, un large éventail de nuances de coloration présentant une résistance à la lumière, au lavage, aux intempéries, à la transpiration et aux différents traitements que peuvent subir les cheveux, particulièrement remarquable.

L'invention porte sur l'utilisation de ces métaphénylènediamines soufrées en position 2 pour la teinture des fibres kératiniques et en particulier les cheveux humains.

Un autre objet de l'invention est constitué par des compositions tinctoriales d'oxydation, destinées à être utilisées pour la teinture des fibres kératiniques et en particulier des cheveux humains, contenant au moins un précurseur de colorant d'oxydation de type ortho et/ou para et au moins certaines métaphénylènediamines soufrées en position 2

Un autre objet de l'invention porte sur le procédé de coloration des fibres kératiniques et en particulier des cheveux humains, mettant en oeuvre une telle composition mélangée à un agent oxydant.

L'invention a également pour objet de nouvelles métaphénylènediamines soufrées en position 2 ainsi que leur procédé de préparation.

D'autres objets de l'invention apparaîtront à la lecture de la description et des exemples qui suivent.

La présente invention a donc pour objet l'utilisation pour la teinture de fibres kératiniques et en particulier les cheveux humains d'au moins, une métaphénylènediamine soufrée en position 2 de formule générale : 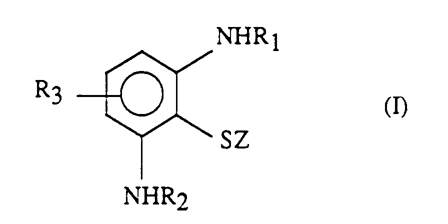 dans laquelle : Z représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical aminoalkyle de formule : 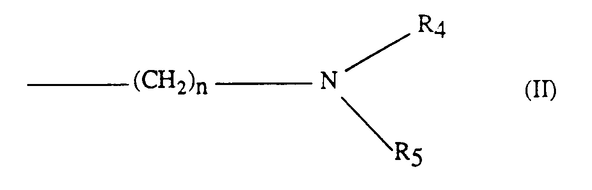 dans laquelle n est un nombre entier compris entre 1 et 6 inclus; R₄ et R₅, identiques ou différents, représentent un atome d'hydrogène ou un radical alkyle en C₁-C₄, hydroxyalkyle en C₁-C₄, acyle en C₁-C₆;
R₁ et R₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkylcarbamyle en C₁-C₆, aminoalkyle en C₁-C₆, acyle en C₁-C₆, carbalcoxy en C₂-C₆, carbamyle ou monoalkyl en C₁-C₆ carbamyle;
R₃ représente un atome d'hydrogène, un radical alkyle en C₁-C₄, nitrile, amide, fluoroalkyle en C₁-C₄, -COOR où R représente un atome d'hydrogène ou un radical alkyle en C₁-C₄; ainsi que les sels d'acides correspondants aux composés de formule (I).

Parmi les significations préférées du radical Z dans les métaphénylènediamines soufrées de formule générale (I) selon l'invention, le radical alkyle en C₁-C₁₈ désigne les radicaux méthyle, éthyle, propyle, butyle, dodécyle, hexadécyle; le radical aralkyle désigne le radical benzyle ; le radical mono ou polyhydroxyalkyle désigne -CH₂-CH₂OH, -CH₂-CHOH-CH₂-OH, -CH₂-CHOH-CH₃; le radical aryle désigne le phényle; le radical aminoalkyle désigne -CH₂-CH₂-NH₂, -CH₂-CH₂-NHCH₃, 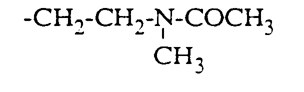 -CH₂-CH₂-NHCOCH₃ ;
lorsque les groupements R₁, R₂, R₄ et R₅ représentent un radical acyle, celui-ci désigne de préférence les radicaux formyle, acétyle et propionyle.
Lorsque le groupement R₃ désigne un radical alkyle, celui-ci est un radical méthyle, éthyle, n-propyle, isopropyle, n-butyle ou isobutyle. Parmi les radicaux fluoroalkyles on peut citer trifluorométhyle et trifluoroéthyle.

Les sels d'acides correspondants aux composés métaphénylènediamines soufrées de formule générale (I) sont choisis de préférence parmi les chlorhydrates, les sulfates ou les bromhydrates.

Parmi les métaphénylènediamines soufrées de formule générale (I), on peut citer :
- le 2-β-acétylaminoéthylthio 1,3-diamino benzène,
- le 2-méthylthio 5-méthyl 1,3-diaminobenzène,
- le 2-méthylthio 5-amido 1,3-diaminobenzène,
- le 2-méthylthio 5-nitrilo 1,3-diaminobenzène,
- le 2-méthylthio 5-trifluorométhyl 1,3-diaminobenzène,
- le 2 méthylthio 1,3-diamino benzène,
- le 2-méthylthio 3-amino 1-carbamoyl-t-butyl benzène,
- le 2-méthylthio 1,3-di(carbamyl-t-butyl) benzène,
- le 2-méthylthio 1,3-diacétylamino benzène,
- le 2-n-propylthio 5-trifluorométhyl 1,3-diaminobenzène,
- le 2-isopropylthio 5-trifluorométhyl 1,3-diaminobenzène,
- le 2-méthylthio 4-méthyl 1,3-diaminobenzène,
- le 2-éthylthio 5-trifluorométhyl 1,3-diaminobenzène,
- le 2-isobutylthio 5-trifluorométhyl 1,3-diaminobenzène,
- le 2-n-butylthio 5-trifluorométhyl 1,3-diaminobenzène,
   et leurs sels d'acides.

Les composés plus particulièrement préférés sont :
- le 2-β-acétylaminoéthylthio 1,3-diamino benzène
- le 2-méthylthio 5-méthyl 1,3-diaminobenzène,
- le 2-méthylthio 5-amido 1,3-diaminobenzène,
- le 2-méthylthio 5-nitrilo 1,3-diaminobenzène,
- le 2-méthylthio 5-trifluorométhyl 1,3-diaminobenzène,
- le 2-méthylthio 1,3-diamino benzène
   et leurs sels d'acides.

Les composés de formule (I) sont utilisables comme coupleurs en présence de précurseurs de colorants d'oxydation de type ortho et/ou para connus en eux-mêmes, permettant de teindre les cheveux par coloration d'oxydation, selon un processus mettant en oeuvre une réaction de condensation oxydative des précurseurs et du coupleur.

Les précurseurs de colorants de type ortho et/ou para sont des composés qui ne sont pas des colorants en eux-mêmes, mais qui forment un colorant par un processus de condensation oxydative, soit sur eux-mêmes, soit en présence d'un coupleur ou modificateur.

Ces précurseurs de colorants d'oxydation de type ortho ou para sont des composés benzéniques ou hétérocycliques qui comportent deux groupements fonctionnels, amino ou hydroxy et amino, en position ortho ou para l'un par rapport à l'autre.

Les précurseurs de colorants d'oxydation de type ortho ou para peuvent être choisis parmi les paraphénylènediamines, les paraaminophénols, les précurseurs hétérocycliques para dérivés de la pyridine ou de la pyrimidine, tels que la 2,5-diaminopyridine, la 2-hydroxy 5-aminopyridine, la 2,4,5,6-tétraaminopyrimidine, la 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (III) : 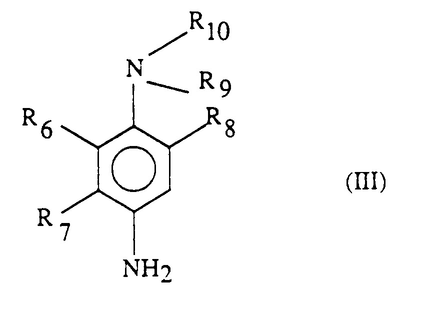 dans laquelle :
R₆, R₇, R₈, identiques ou différents, représentent un atome d'hydrogène ou d'halogène, un radical alkyle, un radical alcoxy, un radical carboxy, sulfo ou hydroxyalkyle en C₁-C₄ ;
R₉ et R₁₀, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino.; ou bien R₉ et R₁₀ forment conjointement avec l'atome d'azote auquel ils sont liés, un hétérocycle pipéridino ou morpholino, sous réserve que R₆ ou R₈ représente un atome d'hydrogène lorsque R₉ et R₁₀ ne représentent pas un atome d'hydrogène, ainsi que les sels de ces composés. Ces radicaux alkyle ou alcoxy ont de préférence 1 à 4 atomes de carbone et désignent notamment les radicaux méthyle, éthyle, propyle, méthoxy et éthoxy

Parmi les composés de formule (III) on peut plus particulièrement citer la paraphénylènediamine, la p-toluylènediamine, la méthoxyparaphénylènediamine, la chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β―hydroxyéthyl)paraphénylènediamine, la 3-méthyl 4-amino N,N-di-(β―hydroxyéthyl)aniline, la 3-chloro 4-amino N,N-di-(β―hydroxyéthyl)aniline, la 4-amino N,N-(éthyl, carbamylméthyl)aniline, la 3-méthyl 4-amino N,N-(éthyl, carbamylméthyl)aniline, la 4-amino N,N-(éthyl, β―pipéridinoéthyl) aniline, la 3-méthyl 4-amino N,N-(éthyl, β―pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl,β-acétylaminoéthyl) aniline, la 4-amino N-(β-méthoxyéthyl) aniline, la 3-méthyl 4-amino N,N-(éthyl, β-acétylaminoéthyl) aniline, la 4-amino N,N-(éthyl, β-mésylaminoéthyl) aniline, la 3-méthyl 4-amino N,N-(éthyl, β-mésylaminoéthyl) aniline, la 4-amino N,N-(éthyl, β― sulfoéthyl)aniline, la 3-méthyl 4-amino N,N-(éthyl, β―sulfoéthyl) aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine, la N,Ndiméthyl 3-méthyl paraphénylènediamine, la N,N-(éthyl,β―hydroxyéthyl)paraphénylènediamine, la N-(dihydroxypropyl)paraphénylènediamine, la N-4'-aminophénylparaphénylènediamine, la N-phénylparaphénylènediamine.

Ces paraphénylènediamines peuvent être utilisées soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β―hydroxyéthyl)4-aminophénol, le 2-méthoxy 4-aminophénol, le 3-méthoxy 4-aminophénol, le 3-(β―hydroxyéthoxy)4-aminophénol, le 2-méthoxyméthyl 4-aminophénol, le 2-aminométhyl 4-aminophénol, le 2-β-hydroxyéthylaminométhyl 4-aminophénol, le 2-éthoxyméthyl 4-aminophénol, le 2-(β-hydroxyéthoxy)méthyl 4-aminophénol.

Les bases dites "doubles" sont des bis-phénylalkylènediamines, répondant à la formule : 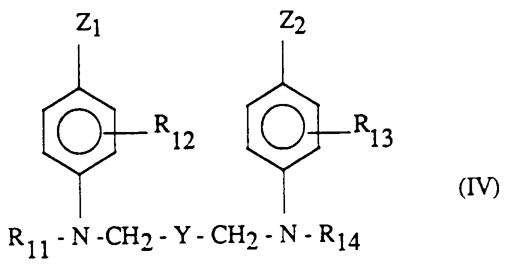 dans laquelle :
Z₁ et Z₂, identiques ou différents, représentent des groupements hydroxyle ou NHR₁₅, où R₁₅ désigne un atome d'hydrogène ou un radical alkyle inférieur ;
R₁₂ et R₁₃, identiques ou différents, représentent soit des atomes d'hydrogène, soit des atomes d'halogène, soit encore des radicaux alkyle ;
R₁₁ et R₁₄, identiques ou différents, représentent un atome d'hydrogène, un radical alkyle, hydroxyalkyle ou aminoalkyle, dont le reste amino peut être substitué; Y représente un radical pris dans le groupe constitué par les radicaux suivants : 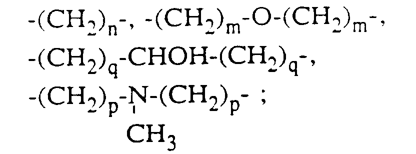 dans lesquels n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (IV) on peut citer le N,N'-bis-(β-hydroéthyl)N,N'-bis-(4'-aminophényl) 1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthylaminophényl)tétraméthylènediamine, la N,N'-bis-(éthyl) N,N'-bis-(4'-amino 3'-méthylphényl)éthylènediamine.

Parmi les orthoaminophénols, on peut citer plus particulièrement le 1-amino-2-hydroxybenzène, le 6-méthyl 1-hydroxy 2-aminobenzène, le 4-méthyl 1-amino 2-hydroxybenzène, le 4-acétylamino 1-amino 2-hydroxybenzène.

Les composés de formule (I) sont appliqués sur les fibres kératininiques et en particulier les cheveux humains au moyen de compositions tinctoriales qui constituent un autre objet de l'invention.

Les compositions conformes à l'invention contiennent dans un milieu approprié pour la teinture au moins une métaphénylènediamine soufrée en position 2 définie ci-dessus. Les compositions préférées contiennent au moins une métaphénylènediamine soufrée en position 2 définie ci-dessus, en association avec au moins un présurseur de colorant d'oxydation tel que défini ci-dessus.

Les compositions tinctoriales conformes à l'invention peuvent également contenir en plus du coupleur répondant à la formule (I) définie ci-dessus, d'autres coupleurs connus en eux-mêmes, tels que les métadiphénols, les métaaminophénols, les métaphénylènediamines différentes de celles de formule (I) ci-dessus, les métaacylaminophénols, les métauréidophénols, les métacarbalcoxyaminophénols, l'α-naphtol, les dérivés indoliques, les coupleurs possédant un groupement méthylène actif, tel que les composés β-cétoniques, les pyrazolones.

Parmi ces coupleurs on peut plus particulièrement citer, le 2, 4-dihydroxyphénoxyéthanol, le 2,4-dihydroxyanisole, le métaaminophénol, le monométhyléther de résorcine, la résorcine, la 2-méthyl-résorcine, le 2-méthyl 5-aminophénol, le 2-méthyl 5-N-(β-hydroxyéthyl) aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-aminobenzomorpholine, le [2―N―(β-hydroxyéthyl) amino 4-amino]―phénoxyéthanol, le 2-amino 4-N-(β-hydroxyéthyl) amino anisole, le (2,4-diamino)phényl-β-γ― dihydroxypropyléther, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chloro résorcinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

On peut rajouter à ces compositions, comme cela est bien connu dans l'état de la technique, notamment en vue de nuancer ou d'enrichir en reflet les colorations apportées par les précurseurs de colorants d'oxydation, des colorants directs tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,3 à 7 % en poids par rapport au poids de la dite composition. La concentration en composés métaphénylènediamines soufrées de formule (I) peut varier entre 0,05 et 3,5 % en poids du poids total de la composition.

Les compositions tinctoriales conformes à l'invention contiennent également dans leur forme de réalisation préférée, des agents tensio-actifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium, les éthanolamides d'acides gras éventuellement oxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5 % en poids par rapport au poids total de la composition.

Le pH de ces compositions est compris entre 4 et 11. Il est ajusté à la valeur désirée à l'aide d'agents alcalinisants bien connus de l'état de la technique, tels que l'ammoniaque, les carbonates alcalins, les alcanolamines tels que les mono-, di- et triéthanolamines ainsi que leurs dérivés ou les hydroxydes de sodium et de potassium, ou d'agents acidifiants classiques, tels que les acides minéraux ou organiques, tels que les acides chlorhydrique, tartrique, citrique, phosporique et sulfonique.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquide, de crème, de gel ou sous toute autre forme appropriée pour réaliser une teinture des fibres kératiniques et notamment des cheveux humains. Ces compositions peuvent être conditionnées en flacons aérosols en présence d'un agent propulseur et former des mousses.

Les composés de formule (I) sont utilisés conformément à l'invention selon un procédé comprenant l'application sur les fibres kératiniques du composé de formule (I) et de précurseurs de colorants d'oxydation ortho et/ou para en présence d'un agent oxydant.

Les compositions tinctoriales conformes à l'invention contenant un précurseur de colorant par oxydation du type para et/ou ortho et un coupleur de formule (I), sont utilisées suivant un procédé mettant en oeuvre la révélation par un agent oxydant.

Conformément à ce procédé on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier les cheveux humains.

Le pH de la composition appliquée sur les cheveux varie de préférence entre 3 et 11. Il est ajusté à la valeur désirée à l'aide d'agents alcalinisants bien connus de l'état de la technique, tels que l'ammoniaque, les carbonates alcalins, les alcanolamines comme la mono-, la di- et la triéthanolamine, ainsi que leurs dérivés ou les hydroxydes de sodium ou de potassium ou d'agents acidifiants classiques, tels que les acides minéraux ou organiques, tels que les acides chlorhydrique, tartrique, citrique, phosphorique et sulfonique. La solution oxydante contient à titre d'agent oxydant, l'eau oxygénée, le peroxyde d'urée, des persels, tels que le persulfate d'ammonium des peracides organiques et leurs sels ou des bromates de métaux alcalins. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on les rince, les lave au shampooing, on les rince à nouveau et on les sèche.

Le coupleur de formule (I) définie ci-dessus, peut également être mis en oeuvre dans un procédé à plusieurs étapes, consistant dans l'une des étapes, à appliquer le précurseur de colorant d'oxydation du type ortho et/ou para ou leur mélange et, dans une autre étape, à appliquer une composition tinctoriale contenant le coupleur de formule (I).

L'agent oxydant peut être introduit, juste avant l'application, dans la composition appliquée dans le deuxième temps ou bien être appliqué sur les fibres kératiniques elles-mêmes, dans un troisième temps, les conditions de pose, de pH, de lavage et de séchage étant identiques à celles indiquées ci-dessus.

Un autre objet de l'invention est constitué par de nouvelles métaphénylènediamines soufrées en position 2 qui répondent à la formule : 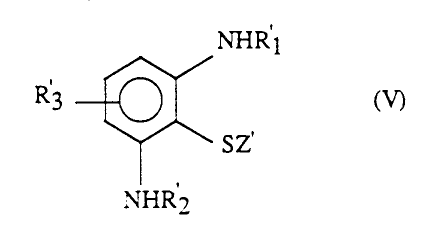 dans laquelle : Z' représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical aminoalkyle de formule : 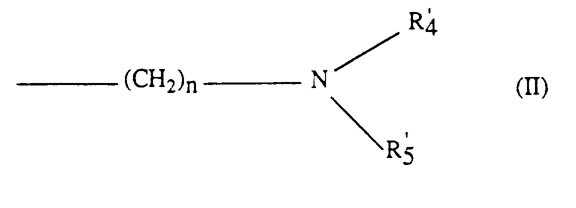 dans laquelle n est un nombre entier compris entre 1 et 6 inclus; R'₄ et R'₅, identiques aux différents, représentent un atome d'hydrogène ou un radical alkyle en C₁-C₄, hydroxyalkyle en C₁-C₄, acyle en C₁-C₆;
R'₁ et R'₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkylcarbamyle en C₁-C₆, aminoalkyle en C₁-C₆, acyle en C₁-C₆, carbalcoxy en C₂-C₆, carbamyle ou monoalkyl en C₁-C₆ carbamyle;
R'₃ représente un atome d'hydrogène, un radical alkyle en C₁-C₄, nitrile, amide, fluoroalkyle en C₁-C₄, -COOR où R représente un atome d'hydrogène ou un radical alkyle en C₁-C₄; ainsi que leurs sels d'acides, sous réserve que :
   · lorsque R'₁ , R'₂ et R'₃ représentent un atome d'hydrogène, Z' est différent du radical alkyle ou du radical aryle;
   · lorsque Z' représente le radical méthyle et R'₃ représente hydrogène, R'₁ et R'₂ ne peuvent représenter simultanément un même radical acyle en C₁-C₆;
   · lorsque Z' représente le radical méthyle et R'₃ représente hydrogène et que l'un des deux radicaux R'₁ et R'₂ représente un atome d'hydrogène, l'autre radical R'₁ ou R'₂ ne peut représenter un radical acyle en C₄;
   · lorsque R'₁ et R'₂ représentent hydrogène et R'₃ représente un radical trifluorométhyle en position 5, Z ne peut représenter un radical alkyle en C₁-C₄;
   · lorsque R'₁ et R'₂ représentent hydrogène et R'₃ représente un radical méthyle en position 4, Z' ne peut désigner méthyle.

Parmi les composés, répondant à la formule (V) définie ci-dessus on peut citer de préférence le 2-β-acétylaminoéthylthio 1,3-diaminobenzène, le 2-méthylthio 5-méthyl 1,3-diaminobenzène, le 2-méthylthio 5-amido 1,3-diaminobenzène et le 2-méthylthio 5-nitrilo 1,3-diaminobenzène.

L'invention a également pour objet les compositions tinctoriales du type définies ci-dessus contenant les composés de formule (V) ainsi que l'utilisation de ces composés pour la teinture des fibres kératiniques.

Les métaphénylènediamines soufrées de formule (V) ou leurs sels peuvent être préparées selon un procédé en plusieurs étapes.

Selon un premier procédé et dans une première étape, on fait réagir en présence d'une base telle que la potasse ou le carbonate de potassium, l'halogéno- 2,6-dinitrobenzène ou un dérivé substitué, sur un thiol de formule (VI) :

Z₁-SH (VI)

dans laquelle Z₁ représente un radical alkyle en C₁-C₁₈, aralkyle dans lequel le radical alkyle est en C₁-C₆, monohydroxyalkyle en C₁-C₆, polyhydroxyalkyle en C₂-C₆, un radical aryle ou un groupement de formule (VII) : 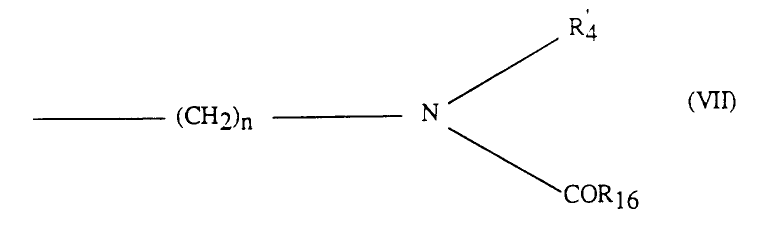 dans laquelle R'₄ et n ont les significations indiquées précédemment dans la formule (V); et
R₁₆ représente un atome d'hydrogène ou un radical alkyle en C₁-C₃ ; dans une deuxième étape, on réduit les substituants nitro du composé de formule (VIII) : 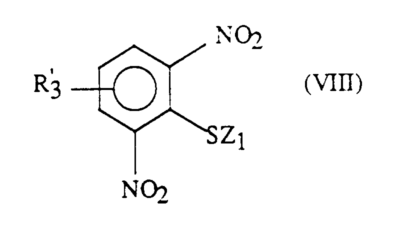 obtenu précédemment pour préparer un composé répondant à la formule (IX) : 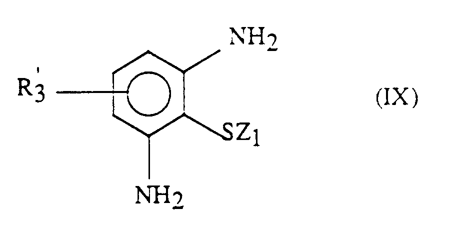 dans laquelle Z₁ a la signification indiquée ci-dessus;
   éventuellement, dans une troisième étape, et suivant la métaphénylènediamine soufrée de formule (I) que l'on souhaite obtenir, on effectue
   a) soit une mono-substitution des amines aromatiques pour obtenir un composé de formule (I) dans laquelle R'₁ et/ou R'₂ sont différents de H
   b) soit une hydrolyse acide du composé de formule (IX) dans laquelle Z₁ représente le groupement de formule (VII) pour obtenir le composé de formule (X) 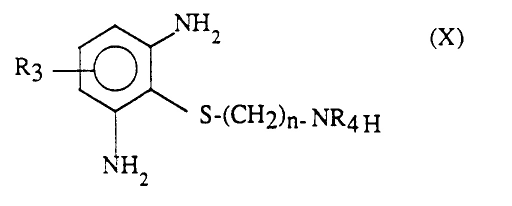 dans laquelle R'₄ et n ont les significations identiques ci-dessus, R'₄ ne désignant toutefois pas de radical acyle en C₁-C₆,
      les amines nucléaires pouvant être ensuite monosubstituées.
   c) soit on effectue au préalable une substitution de l'amine extra-nucléaire du composé de formule (IX) pour obtenir le composé de formule (XI) 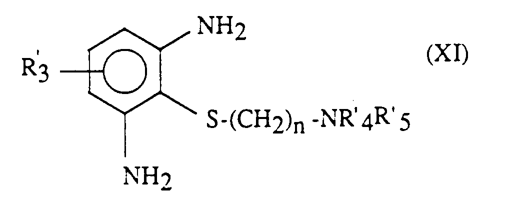 dans laquelle R'₄, R'₅ et n ont les significations indiquées ci-dessus, les amines nucléaires pouvant être ensuite monosubstituées.
Selon un second procédé, et dans une première étape on fait réagir un fluoro nitro benzène substitué de formule (XII) 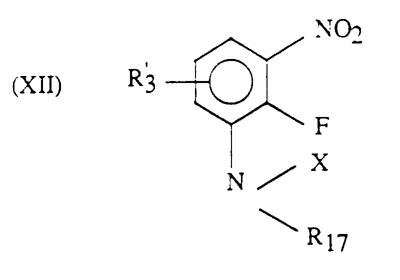 dans laquelle R₁₇ représente un groupement acyle en C₁-C₆ et X représente hydrogène, ou bien R₁₇ et X forment, conjointement avec l'atome d'azote auxquels ils sont liés un cycle oxazolidone, sur un thiol de formule :

Z₁ -SM (XIII)

dans laquelle, M est un alcalin et Z₁ a les significations indiquées ci-dessus.

Dans une deuxième étape, on réduit le substituant nitro du composé de formule (XIV) 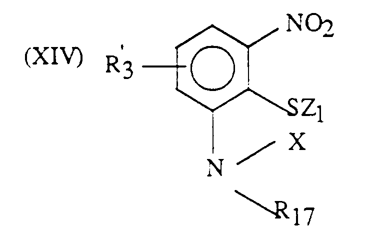 obtenu précédemment pour obtenir le composé de formule (XV) 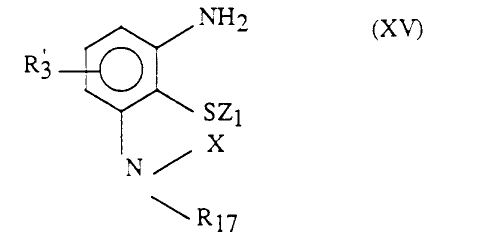 dans laquelle R₁₇ et X ont les significations indiquées ci-dessus ; éventuellement, dans une troisième étape, et suivant la métaphénylènediamine soufrée en position 2 de formule (I) que l'on souhaite obtenir, on effectue une monosubstitution de l'amine aromatique pour obtenir un composé de formule (I) dans laquelle R'₁ ou R'₂ est différent de H.
Enfin, dans une dernière étape, on soumet le composé à une hydrolyse acide, afin de couper le groupement protecteur.

La réduction des groupes nitro s'effectue de préférence en utilisant du fer en milieu acétique ou alors par le cyclohexène en présence d'un catalyseur palladium-charbon ou par tout autre procédé de réduction classique.

La substitution des amines aromatiques ou de l'amine extra-nucléaire peut être effectuée en faisant réagir, par exemple, le bromure d'éthyle, la bromhydrine du glycol, l'éthyl-chloroformiate, la β-chloracétamide, ou l'anhydride acétique.

Les exemples qui suivent sont destinés à illustrer l'invention sans pour autant présenter un caractère limitatif.

### Exemples de Préparation

### Exemple 1 : Préparation du dichlorhydrate de 2-méthylthio 1,3-diamino benzène

A une suspension de 16,8 g (0,24 mole) de thiométhylate de sodium dans 120 ml de diméthoxyéthane, on ajoute goutte à goutte, en 1/2 heure et en maintenant la température entre 20 et 25°C, une solution de 24,3 g (0,12 mole) de 2-chloro 1,3-dinitrobenzène dans 60 ml de diméthoxyéthane. Après trente minutes d'agitation à température ambiante, le milieu réactionnel (suspension brune) est coulé dans 800 ml d'eau glacée.
Le précipité huileux est extrait à l'acétate d'éthyle. Après traitement au charbon CECA et au sulfate de sodium sec, la phase acétate d'éthyle est filtrée sur papier et évaporée à sec sous pression réduite. On obtient une huile orangée (14,8 g) de 2-méthylthio 1,3-dinitrobenzène. Cette huile est mise en solution dans 30 ml d'éthanol à 96° et ajoutée goutte à goutte à une suspension chauffée au reflux de 2,9 g de chlorure d'ammonium et de 63 g de zinc en poudre fine dans 180 ml d'alcool et 26 ml d'eau.
La réaction est exothermique.
Le milieu réactionnel incolore est filtré bouillant. Le filtrat est refroidi à 0°C et est acidifié avec 24 ml d'éthanol absolu chlorhydrique environ 7 N.
Après dilution avec 1,5 ml d'acétone, le dichlorhydrate attendu cristallise.
Les cristaux blancs sont essorés, lavés à l'acétone et séchés sous vide, sur potasse, à 40°C.

On obtient 7,3 g du dichlorhydrate attendu, fondant avec décomposition à 240-245°C et dont l'analyse élémentaire calculée pour C₇H₁₂N₂SCl₂ est

| % | C | H | N | S | Cl |
|---|---|---|---|---|---|
| Calculé | 37,01 | 5,32 | 12,33 | 14,12 | 31,21 |
| Trouvé | 37,06 | 5,32 | 12,35 | 14,10 | 31,33 |

### Exemple 2 : Préparation du dichlorhydrate du 2-β-acétylaminoéthylthio 1,3-diaminobenzène

### 1ère étape : Synthèse du 2-β-acétylaminoéthylthio 1,3-dinitrobenzène.

On dissout 10 g de potasse en poudre dans une solution de 35,7 g (0,3 mole) de N-(2-mercaptoéthyl)-acétamide dans 100 ml de diméthoxyéthane chauffée à 45°C.
Après refroidissement à 15°C on coule, goutte à goutte en 30 mn, une solution de 24,3 g (0,12 mole) de 2-chloro-1,3-dinitrobenzène dans 50 ml de diméthoxyéthane.
On maintient la température entre 15 et 20°C pendant 30 mn supplémentaires.
Le milieu réactionnel est versé dans 300 ml d'eau glacée.
Le précipité cristallisé jaune orangé est essoré, réempaté dans l'eau et séché sous vide à 30°C sur anhydride phosphorique.
Après recristallisation de l'éthanol à 96°, on obtient 15,7 g de cristaux jaune fondant à 110°C et dont l'analyse élémentaire calculée pour C₁₀H₁₁N₃O₅ S est

| % | C | H | N | O | S |
|---|---|---|---|---|---|
| Calculé | 42,10 | 3,89 | 14,73 | 28,04 | 11,24 |
| Trouvé | 42,32 | 3,93 | 14,68 | 27,84 | 11,18 |

### 2ème étape : Réduction

La réduction du composé obtenu ci-dessus à la 1ère étape est effectuée selon le mode opératoire décrit pour l'exemple 1.
Le dichlorhydrate obtenu est purifié par recristallisation d'un mélange eau-éthanol (1/6) au reflux.
On obtient des cristaux blancs fondant avec décomposition à 197-200°C et dont l'analyse élémentaire calculée pour C₁₀H₁₇N₃OS Cl₂ est:

| % | C | H | N | O | S | Cl |
|---|---|---|---|---|---|---|
| Calculé | 40,27 | 5,75 | 14,09 | 5,36 | 10,75 | 23,78 |
| Trouvé | 40,08 | 5,80 | 14,06 | 5,85 | 10,68 | 23,60 |

### Exemple 3 : Préparation du dichlorhydrate de 2-méthylthio 5-méthyl 1,3-diaminobenzène

1ère étape : Synthèse du 2-méthylthio 5-méthyl 1,3-dinitrobenzène A une suspension de 25 g (0,35 mole) de thiométhylate de sodium dans 250 ml de diméthoxyéthane, on ajoute goutte à goutte, en une heure et en maintenant la température entre 17°C et 20°C, une solution de 50,0 g (0,231 mole) de 2-chloro 5-méthyl 1,3-dinitrobenzène dans 140 ml de diméthoxyéthane.

Après une heure d'agitation à température ambiante, le milieu réactionnel est coulé dans 1,5 l d'eau glacée.

Le précipité cristallisé est essoré, réempâté dans l'eau, séché puis recristallisé de l'acétate d'éthyle bouillant.

On obtient 22,2 g de cristaux jaune pâle fondant à 106°C et dont l'analyse élémentaire calculée pour C₈H₈N₂O₄S est :

| % | C | H | N | O | S |
|---|---|---|---|---|---|
| Calculé | 42,10 | 3,53 | 12,27 | 28,04 | 14,05 |
| Trouvé | 42,18 | 3,59 | 12,18 | 28,25 | 14,11 |

### 2ème étape : Réduction

A une suspension, chauffée au reflux, de 4,4 g de chlorure d'ammonium et de 82 g de zinc en poudre fine dans 280 ml d'alcool et 33 ml d'eau, on ajoute, par portions, de façon à maintenir le reflux sans chauffage, 20,5 g (0,09 mole) du composé dinitré ci-dessus obtenu à la première étape.
La réaction est exothermique.
Le milieu réactionnel est filtré bouillant, évaporé en partie de façon à éliminer l'alcool et extrait à l'éther éthylique.

Après séchage sur sulfate de sodium et filtration, la phase éthérée est acidifiée avec 30 ml d'éthanol absolu chlorhydrique environ 6 N.

Le précipité de dichlorhydrate de 2-méthylthio 5-méthyl 1,3-diaminobenzène est essoré et séché sur potasse.

On obtient 15,2 g de cristaux blancs recristallisés d'un mélange éthanol/eau fondant avec décomposition à 220-224°C et dont l'analyse élémentaire calculée pour C₈H₁₂N₂S,2HCl est :

| % | C | H | N | S | Cl |
|---|---|---|---|---|---|
| Calculé | 39,84 | 5,85 | 11,61 | 13,29 | 29,40 |
| Trouvé | 39,65 | 5,89 | 11,81 | 13,20 | 29,30 |

### Exemple 4 : Préparation du dichlorhydrate de 2-méthylthio 5-amido 1,3-diaminobenzène.

1ère étape : Synthèse du 2-méthylthio 5-amido 1,3-dinitrobenzène Ce composé est préparé selon le mode opératoire décrit pour l'exemple 3, étape 1.
A partir de 24,6 g (0,10 mole) de 2-chloro 5-amido 1,3-dinitrobenzène, on obtient 5,8 g de cristaux jaune recristallisés de l'éthanol à 96°, fondant à 196 °C et dont l'analyse élémentaire calculée pour C₈H₇N₃O₅S est :

| % | C | H | N | O | S |
|---|---|---|---|---|---|
| Calculé | 37,36 | 2,74 | 16,34 | 31,10 | 12,47 |
| Trouvé | 37,55 | 2,76 | 16,55 | 31,27 | 12,56 |

### 2ème étape : Réduction

La réduction est effectuée selon le mode opératoire décrit pour l'exemple 3, étape 2.

A partir de 12,5 g (0,0486 mole) du composé dinitré obtenu à la première étape, on obtient 3,6 g de cristaux blancs, recristallisés d'un mélange eau/éthanol, de dichlorhydrate de 2-méthylthio 5-amido 1,3-diaminobenzène, fondant avec décomposition à 230-232 °C et dont l'analyse élémentaire calculée pour C₈H₁₁N₃OS, 2 HCl est :

| % | C | H | N | O | S | Cl |
|---|---|---|---|---|---|---|
| Calculé | 35,56 | 4,85 | 15,55 | 5,92 | 11,87 | 26,24 |
| Trouvé | 35,80 | 4,82 | 15,43 | 6,19 | 12,07 | 26,36 |

### Exemple 5 : Préparation du monochlorhydrate de 2-méthylthio 5-nitrilo 1,3-diaminobenzène

1ère étape : Synthèse du 2-méthylthio 5-nitrilo 1,3-diaminobenzène Ce composé est préparé selon le mode opératoire décrit pour l'exemple 3, étape 1.

A partir de 7,5 g (0,033 mole) de 2-chloro 5-nitrile 1,3-dinitrobenzène, on obtient 3,7 g de cristaux jaune, recristallisés de l'acétate d'isopropyle au reflux, fondant à 137°C et dont l'analyse élémentaire calculée pour C₈H₅N₃O₄S est :

| % | C | H | N | O | S |
|---|---|---|---|---|---|
| Calculé | 40,17 | 2,11 | 17,57 | 26,75 | 13,40 |
| Trouvé | 40,29 | 2,17 | 17,59 | 26,93 | 13,45 |

### 2ème étape : Réduction

La réduction est effectuée selon le procédé décrit pour l'exemple 3, étape 2.

A partir de 3,5 g (0,0146 mole) du composé dinitré obtenu à la première étape, on obtient après recristallisation d'un mélange éthanol/eau, 1,3 g de cristaux blancs de monochlorhydrate de 2-méthylthio 5-nitrilo 1,3-diaminobenzène fondant avec décomposition à 198-200°C et dont l'analyse élémentaire calculée pour C₈H₉N₃S, HCl est :

| % | C | H | N | S | Cl |
|---|---|---|---|---|---|
| Calculé | 44,55 | 4,67 | 19,48 | 14,86 | 16,44 |
| Trouvé | 44,66 | 4,76 | 19,64 | 15,00 | 16,43 |

### Teinture à pH acide

### Composition A

| | |
|---|---|
| · Alcool oléique polyglycérolé à 2 moles de glycérol | 4,0 g |
| · Alcool oléique polyglycérolé à 4 moles de glycérol à 78 % de MA | 5,69 g MA |
| · Acide oléique | 3,0 g |
| · Amine oléique à 2 moles d'oxyde d'éthylène vendue sous | |
| la dénomination ETHOMEEN 012 par la Société AKZO | 7,0 g |
| · Laurylamino succinamate de diéthylaminopropyle, | |
| sel de sodium à 55 % de MA | 3,0 g MA |
| · Alcool oléique | 5,0 g |
| · Diéthanolamide d'acide oléique | 12,0 g |
| · Propylèneglycol | 3,5 g |
| · Alcool éthylique | 7,0 g |
| · Dipropylèneglycol | 0,5 g |
| · Monométhyléther de propylèneglycol | 9,0 g |
| · Métabisulfite de sodium en solution aqueuse à 35 % | 0,455 g |
| · Acétate d'ammonium | 0,8 g |
| · Antioxydant, séquestrant qs | |
| · Parfum, conservateurs qs | |
| · Monoéthanolamine qsp pH 9,8 | |
| · Colorants | x g |
| · Eau déminéralisée qsp | 100,0 g |

### Composition B

Elle est constituée par une solution d'eau oxygénée à 20 volumes dont le pH et ajusté entre 1 et 1,5 par de l'acide orthophosphorique.

### Protocole de teinture

La composition A est mélangée, poids pour poids, avec la composition B d'eau oxygénée. Le mélange obtenu est appliqué sur des cheveux gris naturels à 90 % de blancs, permanentés ou non, pendant 30 minutes. Les cheveux sont ensuite rincés, lavés au shampooing, puis rincés à nouveau et séchés.

| **EXEMPLES** | **1** | **2** | **3** | **4** | **5** |
|---|---|---|---|---|---|
| Composition A contenant : | | | | | |
| -2-β-acétylaminoéthylthio 1,3-diaminobenzène, dichlorhydrate | 0,89 g | | | | |
| -2-méthylthio 1,3-diaminobenzène, dichlorhydrate | | 0,68 g | | | |
| 2-méthylthio 5-méthyl 1,3-diaminobenzène, dichlorhydrate | | | 0,724 g | | |
| 2-méthylthio 5-nitrilo 1,3-diaminobenzène, monochlorhydrate | | | | 0,080 g | |
| 2-méthylthio 5-trifluorométhyl 1,3-diaminobenzène, monochlorhydrate | | | | | 0,050 g |
| -2,6-diméthylparaphénylènediamine | 0,41 g | 0,41 g | | | |
| Paraphénylènediamine | | | 0,324 g | 0,324 g | |
| 2,4-diaminophénoxy éthanol, dichlorhydrate | | | | 0,651 g | 0,675 g |
| 2,6-diméthylparaphénylènediamine, dichlorhydrate | | | | | 0,627 g |
| Mélange poids pour poids de A et B pH du mélange | 6,4 | 6,3 | 6,4 | 6,5 | 6,3 |
| Nuances obtenues · sur cheveux gris naturels à 90 % de blancs | bleu légèrement vert | | acajou doré | | bleu moyen |
| · sur cheveux gris naturels à 90 % de blancs permanentés | | bleu nuit | | bleu noir profond | |

### Teinture à pH basique

### Composition C

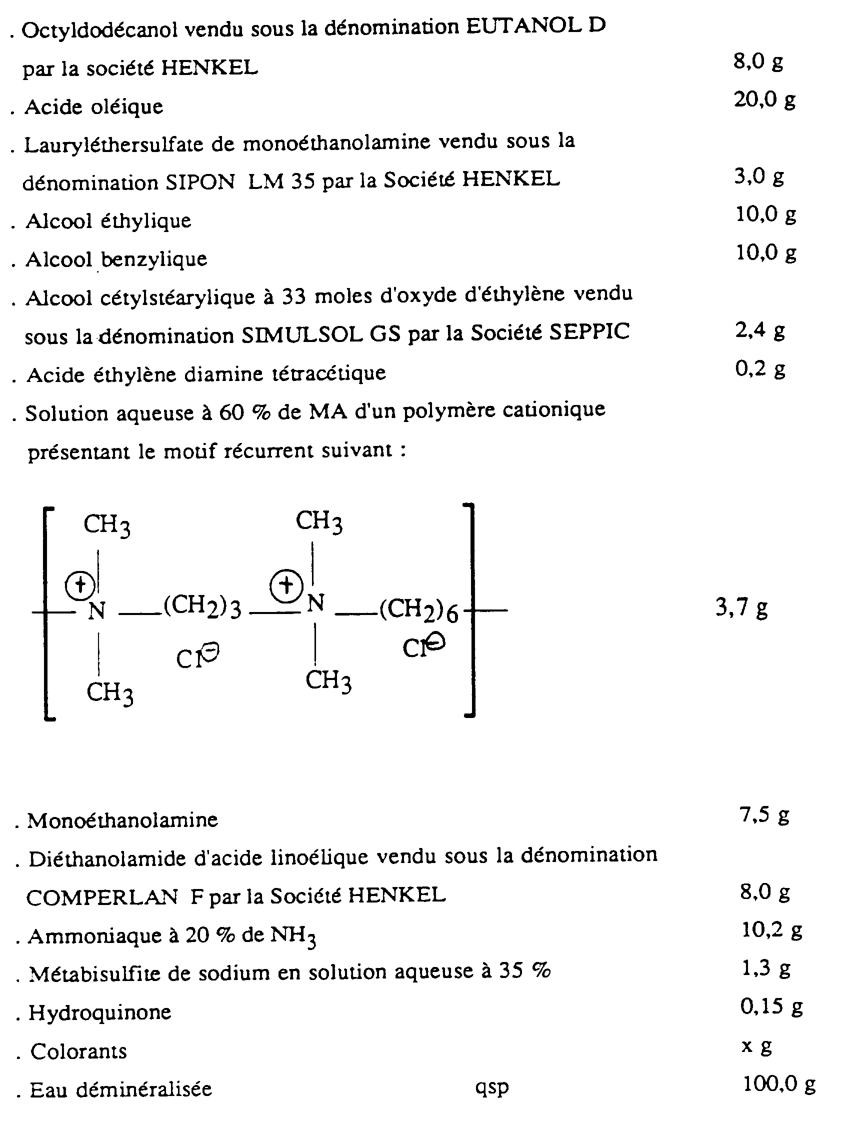

### Composition D

Eau oxygénée à 20 volumes et à pH = 3.

### Protocole de teinture

La composition C est mélangée, poids pour poids, avec la composition D. Le mélange obtenu est appliqué sur des cheveux gris naturels à 90 % de blancs, permanentés ou non, pendant 30 mn. Les cheveux sont ensuite rincés, lavés au shampooing, puis rincés à nouveau et séchés.

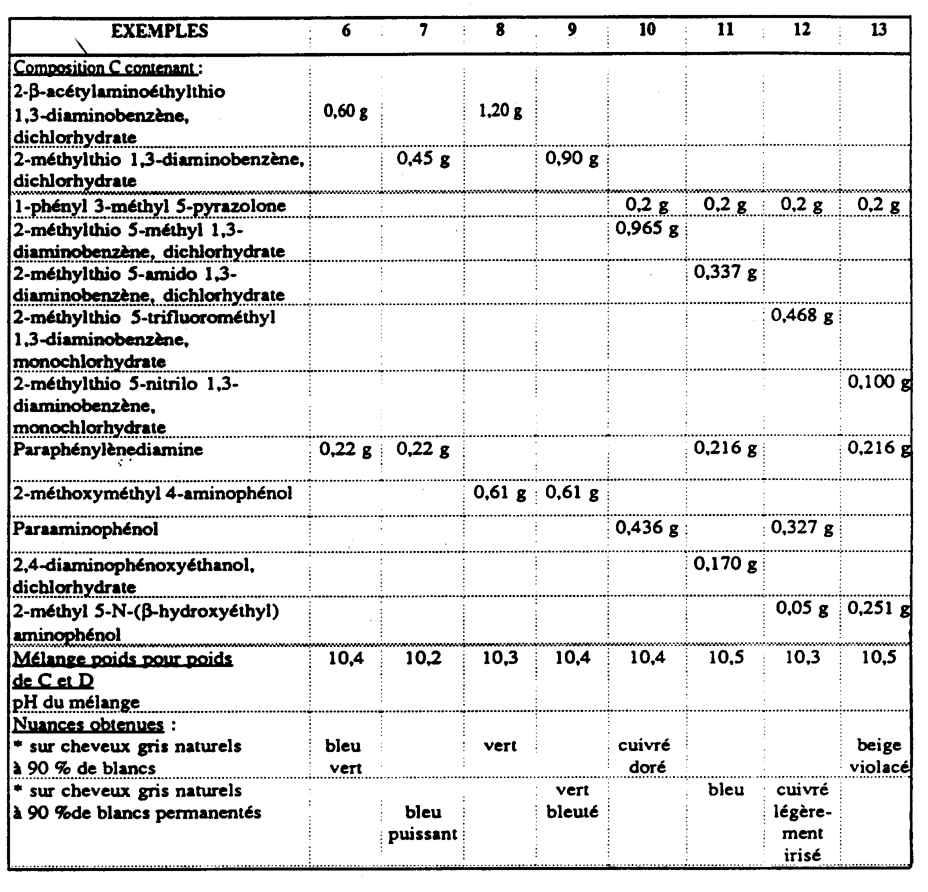

## Revendications

1. Utilisation pour la teinture de fibres kératiniques, et en particulier les cheveux humains, d'au moins une métaphénylènediamine soufrée en position 2 de formule générale : 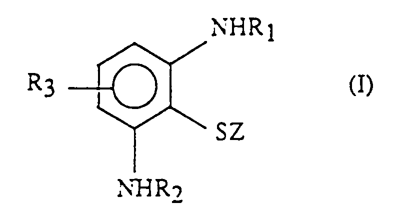 dans laquelle : Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 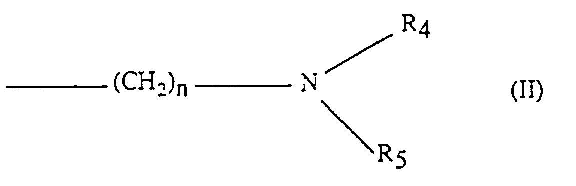 dans laquelle n est un nombre entier compris entre 1 et 6 inclus, R₄ et R₅, identiques ou différents, représentent un atome d'hydrogène ou un radical alkyle en C₁-C₄, hydroxyalkyle en C₁-C₄, acyle en C₁-C₆;
R₁ et R₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkylcarbamyle en C₁-C₆, aminoalkyle en C₁-C₆, acyle en C₁-C₆, carbalcoxy en C₂-C₆, carbamyle ou monoalkyl en C₁-C₆ carbamyle;
R₃ représente un atome d'hydrogène, un radical alkyle en C₁-C₄, nitrile, amide, fluoroalkyle en C₁-C₄, -COOR où R représente un atome d'hydrogène ou un radical alkyle en C₁-C₄; ainsi que les sels d'acides correspondants aux composés de formule (I).

2. Utilisation selon la revendication 1, caractérisée par le fait que la métaphénylènediamine soufrée en position 2 de formule (I) est mise en oeuvre en présence de précurseurs de colorants d'oxydation de type ortho et/ou para.

3. Utilisation selon la revendication 1 ou 2, caractérisée par le fait que dans le composé de formule (I), Z désigne un radical méthyle, éthyle, propyle, butyle, dodécyle, hexadécyle, le radical benzyle, le radical phenyle ou un radical mono ou polyhydroxyalkyle choisi parmi:
-CH₂-CH₂-OH, -CH₂-CHOH-CH₂-OH, -CH₂-CHOH-CH₃, aminoalkyle choisi parmi
-CH₂-CH₂NH₂, -CH₂-CH₂-NHCH₃, 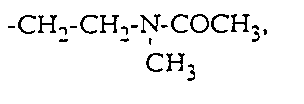 -CH₂-CH₂-NH-COCH₃ ;
et lorsque R₁, R₂, R₄ ou R₅ désignent un radical acyle, celui-ci représente un groupement formyle, acétyle ou propionyle, et R₃ est tel que défini dans la revendication 1.

4. Utilisation selon l'une quelconque des revendications 1 à 3 caractérisée en ce que les composés de formule (I) sont choisis parmi les composés suivants :
- le 2-β-acétylaminoéthylthio 1,3-diamino benzène,
- le 2-méthylthio 5-méthyl 1,3-diaminobenzène,
- le 2-méthylthio 5-amido 1,3-diaminobenzène,
- le 2-méthylthio 5-nitrilo 1,3-diaminobenzène,
- le 2-méthylthio 5-trifluorométhyl 1,3-diaminobenzène,
- le 2 méthylthio 1,3-diamino benzène,
- le 2-méthylthio 3-amino 1-carbamyl-t-butyl benzène,
- le 2-méthylthio 1,3-di(carbamyl-t-butyl) benzène,
- le 2-méthylthio 1,3-diacétylamino benzène,
- le 2-n-propylthio 5-trifluorométhyl 1,3-diaminobenzène,
- le 2-isopropylthio 5-trifluorométhyl 1,3-diaminobenzène,
- le 2-méthylthio 4-méthyl 1,3-diaminobenzène,
- le 2-éthylthio 5-trifluorométhyl 1,3-diaminobenzène,
- le 2-isobutylthio 5-trifluorométhyl 1,3-diaminobenzène,
- le 2-n-butylthio 5-trifluorométhyl 1,3-diaminobenzène, et leurs sels d'acides.

5. Utilisation selon l'une quelconque des revendications 1 à 4, caractérisé par le fait que les sels d'acides correspondants sont choisis parmi les chlorhydrates, les sulfates, ou les bromhydrates.

6. Utilisation selon l'une quelconque des revendications 2 à 5, caractérisée par le fait que les précurseurs de colorants d'oxydation sont choisis parmi les paraphénylènediamines, les paraaminophénols, les précurseurs hétérocycliques para dérivés de la pyridine ou de la pyrimidine, les orthoaminophénols et les bis-phénylalkylènediamines.

7. Composition tinctoriale pour fibres kératiniques, et en particulier les cheveux humains, contenant dans un milieu approprié pour la teinture, au moins un précurseur de colorant d'oxydation de type ortho et/ou para, et au moins, à titre de coupleur, une métaphénylènediamine soufrée en position 2 telle que définie dans les revendications 1, 3 ou 4.

8. Composition tinctoriale selon la revendication 7, caractérisée en ce qu'elle contient des coupleurs tels que les métadiphénols, des métaaminophénols, des métaphénylènediamines différentes de celles de formule (I), des métaacylaminophénols, des métauréidophénols, des métacarbalcoxyaminophénols, l'α-naphtol, les dérivés indoliques, les coupleurs possédant un groupe méthylène actif.

9. Composition tinctoriale selon l'une quelconque des revendications 7 à 8, caractérisée en ce qu'elle contient 0, 05 à 3,5 % en poids du poids total de la composition d'au moins un composé de formule (I).

10. Composition tinctoriale selon l'une quelconque des revendications 7 à 9, caractérisée en ce qu'elle contient de 0,3 à 7 % en poids par rapport au poids total de la composition, de précurseurs de colorants d'oxydation de type ortho et/ou para et de coupleurs.

11. Composition tinctoriale selon l'une quelconque des revendications 7 à 10, caractérisée en ce qu'elle contient des agents tensio-actifs cationiques, anioniques, non-ioniques, amphotères ou leurs mélanges, en des concentrations comprises entre 0,5 % et 55 % en poids par rapport au poids total de la composition, des solvants organiques en des concentrations comprises entre 1 et 40 % en poids par rapport au poids total de la composition, des colorants directs, des agents épaississants en des concentrations comprises entre 0,1 et 5 % en poids par rapport au poids total de la composition et des agents antioxydants en des proportions comprises entre 0,05 et 1,5 % en poids par rapport au poids total de la composition.

12. Composition tinctoriale selon l'une quelconque des revendications 7 à 11, caractérisée en ce qu'elle se présente sous forme de liquide, de crème, de gel ou sous toute autre forme appropriée, ou peut être conditionnée en flacon aérosol en présence d'un agent propulseur et former des mousses.

13. Procédé de teinture d'oxydation des fibres kératiniques et en particulier des cheveux humains, caractérisé en ce que l'on applique sur, les fibres kératiniques au moins un précurseur de colorant d'oxydation de type ortho et/ou para, et une métaphénylènediamine soufrée en position 2 de formule générale : 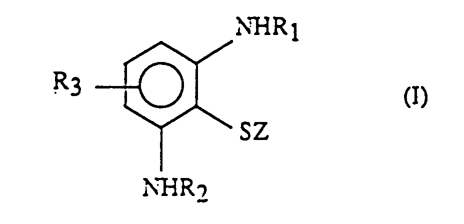 dans laquelle : Z représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical aminoalkyle de formule : 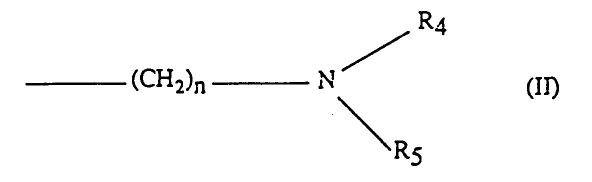 dans laquelle n est un nombre entier compris entre 1 et 6 inclus, R₄ et R₅, identiques ou différents, représentent un atome d'hydrogène ou un radical alkyle en C₁-C₄, hydroxyalkyle en C₁-C₄, acyle en C₁-C₆;
R₁ et R₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkylcarbamyle en C₁-C₆, aminoalkyle en C₁-C₆, acyle en C₁-C₆, carbalcoxy en C₂-C₆, carbamyle ou monoalkyl en C₁-C₆ carbamyle;
R₃ représente un atome d'hydrogène, un radical alkyle en C₁-C₄, nitrile, amide, fluoroalkyle en C₁-C₄, -COOR où R représente hydrogène ou un radical alkyle en C₁-C₄, ainsi que les sels d'acides correspondants aux composés de formule (I), en présence d'un agent oxydant.

14. Procédé selon la revendication 13, caractérisé en ce que l'on mélange, au moment de l'emploi, une composition tinctoriale pour fibres kératiniques, et en particulier les cheveux humains, contenant, dans un milieu approprié pour la teinture, au moins un précurseur de colorant d'oxydation de type ortho et/ou para, et au moins, à titre de coupleur, une métaphénylènediamine soufrée en position 2 telle que définie dans la revendication 13, avec une solution oxydante en une quantité suffisante pour développer la coloration, la composition résultante ayant un pH variant entre 3 et 11, et on applique le mélange ainsi obtenu sur les fibres kératiniques, et en particulier les cheveux humains.

15. Procédé selon la revendication 13, caractérisé en ce que l'on applique dans un premier temps le précurseur de colorant d'oxydation de type ortho et/ou para ou leur mélange sur les fibres kératiniques, dans un deuxième temps une composition contenant le coupleur de formule (I), et l'on développe la coloration à l'aide d'un agent oxydant, présent dans ladite composition ou bien appliqué sur les fibres kératiniques dans un troisième temps.

16. Procédé de teinture d'oxydation selon l'une quelcenque des revendications 13 à 15, caractérisé par le fait qu'on laisse poser pendant 10 à 40 minutes, de préférence 15 à 30 minutes, on rince les cheveux, on les lave au shampooing, on les rince à nouveau et on les sèche.

17. Métaphénylènediamine soufrée en position 2 de formule générale : 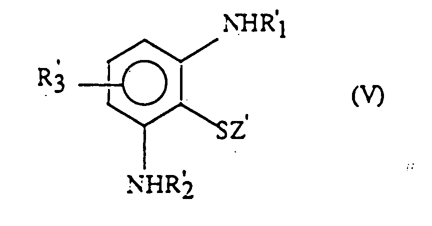 dans laquelle, Z' désigne un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule: 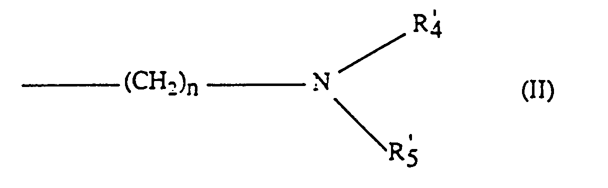 dans laquelle n est un nombre entier compris entre 1 et 6 inclus; R'₄ et R'₅, identiques ou différents, représentent un atome d'hydrogène, un radical alkyle en C₁-C₄, hydroxyalkyle en C₁-C₄ ou acyle en C₁-C₆ :
R'₁ et R'₂, identiques ou différents, désignent un atome d'hydrogène, un radical alkyle en C₁-C₆, monohydroxyalkyle en C₁-C₆, polyhydroxyalkyle en C₂-C₆, monocarbamylalkyle en C₁-C₆, dialkylcarbamyle en C₁-C₆, aminoalkyle en C₁-C₆, acyle en C₁-C₆, carbalcoxy en C₂-C₆, carbamyle ou monoalkyl en C₁-C₆ carbamyle;
R'₃ représente un atome d'hydrogène, un radical alkyle en C₁-C₄, nitrile, amide, fluoroalkyle en C₁-C₄, -COOR où R représente un atome d'hydrogène ou un radical alkyle en C₁-C₄;
ainsi que leurs sels d'acides
sous réserve que :
· lorsque R'₁ et R'₂ et R'₃ représentent un atome d'hydrogène, Z' est différent du radical alkyle ou du radical aryle;
· lorsque Z' représente le radical méthyle et R'₃ représente un atome d'hydrogène R'₁ et R'₂ ne peuvent représenter simultanément un même radical acyle en C₁-C₆;
· lorsque Z' représente le radical méthyle et que R'₃ représente un atome d'hydrogène et que l'un des deux radicaux R'₁ et R'₂ représente un atome d'hydrogène, l'autre radical R'₁ ou R'₂ ne peut représenter un radical acyle en C₄;
· lorsque R'₁ et R'₂ représentent un atome d'hydrogène et R'₃ un radical trifluorométhyle en position 5, Z' ne peut représenter un radical alkyle
· lorsque R'₁ et R'₂ représentent un atome d'hydrogène et R'₃ représente le radical méthyle en position 4, Z' ne peut représenter un radical méthyle.

18. Métaphénylènediamine soufrée en position 2 selon la revendication 17, caractérisée par le fait qu'elle est choisie parmi le 2-β-acétylaminoéthylthio 1,3-diaminobenzène, le 2-méthylthio 5-méthyl 1,3-diaminobenzène, le 2-méthylthio 5-amido 1,3-diaminobenzène, et le 2-méthylthio 5-nitrilo 1,3-diaminobenzène.

19. Composition tinctoriale pour fibres kératiniques et en particulier les cheveux humains, caractérisée par le fait qu'elle contient, dans un milieu approprié pour la teinture, au moins une métaphénylènediamine soufrée en position 2 de formule (V) telle que définie dans l'une des revendications 17 ou 18.

## Claims

1. Use for dyeing keratinous fibers, and in particular human hair, of at least one 2-sulfurated meta-phenylenediamine of general formula: 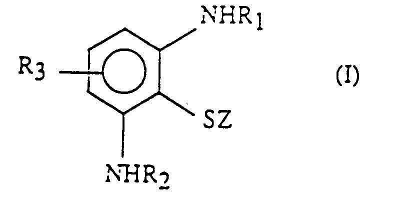 in which: Z represents a C₁-C₁₈ alkyl radical, an aralkyl radical in which the alkyl radical is C₁-C₆, a C₁-C₆ monohydroxyalkyl or C₂-C₆ polyhydroxyalkyl radical, an aryl radical or an aminoalkyl radical of formula: 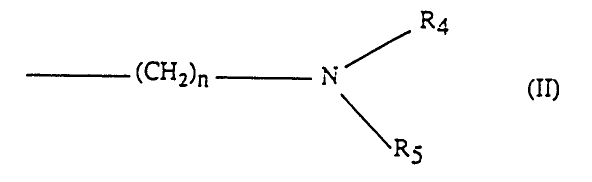 in which n is an integer between 1 and 6 inclusive and R₄ and R₅, which are identical or different, represent a hydrogen atom or a C₁-C₄ alkyl, C₁-C₄ hydroxyalkyl or C₁-C₆ acyl radical;
R₁ and R₂, which are identical or different, represent a hydrogen atom, a C₁-C₆ alkyl radical, a C₁-C₆ monohydroxyalkyl radical, a C₂-C₆ polyhydroxyalkyl radical, a C₁-C₆ monocarbamoylalkyl radical, a C₁-C₆ dialkylcarbamoyl radical, a C₁-C₆ aminoalkyl radical, a C₁-C₆ acyl radical, a C₂-C₆ carbalkoxy radical, a carbamoyl radical or a C₁-C₆ monoalkylcarbamoyl radical;
R₃ represents a hydrogen atom, a C₁-C₄ alkyl radical, a nitrile radical, an amide radical, a C₁-C₄ fluoroalkyl radical or a radical -COOR where R represents a hydrogen atom or a C₁-C₄ alkyl radical, and the acid salts corresponding to the compounds of formula (I).

2. Use according to claim 1, characterized in that the 2-sulfurated meta-phenylenediamine of formula (I) is used in the presence of oxidation dye precursors of ortho and/or para type.

3. Use according to claim 1 or 2, characterized in that, in the compound of formula (I), Z denotes a methyl, ethyl, propyl, butyl, dodecyl or hexadecyl radical, the benzyl radical, the phenyl radical or a mono- or polyhydroxyalkyl radical chosen from:
-CH₂-CH₂-OH, -CH₂-CHOH-CH₂-OH, -CH₂-CHOH-CH₃, or an aminoalkyl radical chosen from
-CH₂-CH₂-NH₂, -CH₂-CH₂-NHCH₃, 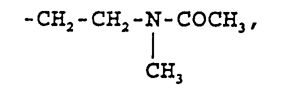 -CH₂-CH₂-NHCOCH₃;
and when R₁, R₂, R₄ or R₅ denote an acyl radical, the latter represents a formyl, acetyl or propionyl group, and R₃ is as defined in claim 1.

4. Use according to any one of claims 1 to 3, characterized in that the compounds of formula (I) are chosen from the following compounds:
- 2-β-acetylaminoethylthio-1,3-diaminobenzene,
- 2-methylthio-5-methyl-1,3-diaminobenzene,
- 2-methylthio-5-amido-1,3-diaminobenzene ,
- 2-methylthio-5-nitrilo-1,3-diaminobenzene ,
- 2-methylthio-5-trifluoromethyl-1,3-diaminobenzene,
- 2-methylthio-1,3-diaminobenzene,
- 2-methylthio-3-amino-1-(carbamoyl-t-butyl)benzene
- 2-methylthio-1,3-di(carbamoyl-t-butyl)benzene ,
- 2-methylthio-1,3-diacetylaminobenzene,
- 2-n-propylthio-5-trifluoromethyl-1,3-diaminobenzene,
- 2-isopropylthio-5-trifluoromethyl-1,3-diaminobenzene,
- 2-methylthio-4-methyl-1,3-diaminobenzene,
- 2-ethylthio-5-trifluoromethyl-1,3-diaminobenzene,
- 2-isobutylthio-5-trifluoromethyl-1,3-diaminobenzene,
- 2-n-butylthio-5-trifluoromethyl-1,3-diaminobenzene,
and their acid salts.

5. Use according to any one claims 1 to 4, characterized in that the corresponding acid salts are chosen from hydrochlorides, sulfates or hydrobromides.

6. Use according to any one of claims 2 to 5, characterized in that the oxidation dye precursors are chosen from paraphenylenediamines, paraaminophenols, para heterocyclic precursors derived from pyridine or pyrimidine, orthoaminophenols and bisphenylalkylenediamines.

7. Dyeing composition for keratinous fibers, and in particular human hair, containing, in a medium suitable for dyeing, at least one oxidation dye precursor of ortho and/or para type and at least, as coupler, one 2-sulfurated metaphenylenediamine as defined in claims 1, 3 or 4.

8. Dyeing composition according to claim 7, characterized in that it contains couplers such as metadiphenols, metaaminophenols, metaphenylenediamines other than those of formula (I), metaacylaminophenols, metaureidophenols, metacarbalkoxyaminophenols, α-naphthol, indole derivatives or couplers having an active methylene group.

9. Dyeing composition according to one of claims 7 to 8, characterized in that it contains 0.05 to 3.5 % by weight of the total weight of the composition of at least one compound of formula (I).

10. Dyeing composition according to any one of claims 7 to 9, characterized in that it contains from 0.3 to 7 % by weight, with respect to the total weight of the composition, of oxidation dye precursors of ortho and/or para type and of couplers.

11. Dyeing composition according to any one of claims 7 to 10, characterized in that it contains cationic, anionic, nonionic or amphoteric surface-active agents or their mixtures, in concentrations of between 0.5 % and 55 % by weight with respect to the total weight of the composition, organic solvents in concentrations of between 1 and 40 % by weight with respect to the total weight of the composition, direct dyes, thickening agents in concentrations of between 0.1 and 5 % by weight with respect to the total weight of the composition and antioxidizing agents in proportions of between 0.05 and 1.5 % by weight with respect to the total weight of the composition.

12. Dyeing composition according to any one of claims 7 to 11, characterized in that it is provided in the liquid, cream or gel form or any other suitable form, or can be packaged in an aerosol container in the presence of a propellant and can form foams.

13. Oxidation dyeing process for keratinous fibers and in particular human hair, characterized in that there is applied to keratinous fibers at least one oxidation dye precursor of ortho and/or para type and one 2-sulfurated metaphenylenediamine of general formula: 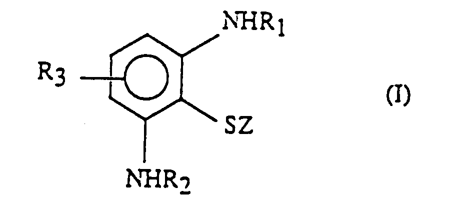 in which: Z represents a C₁-C₁₈ alkyl radical, an aralkyl radical in which the alkyl radical is C₁-C₆, a C₁-C₆ monohydroxyalkyl or C₂-C₆ polyhydroxyalkyl radical, an aryl radical or an aminoalkyl radical of formula: 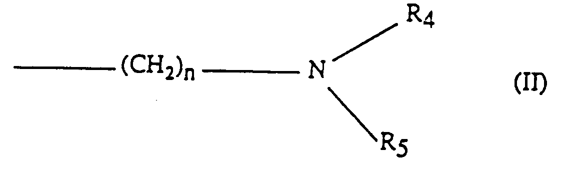 in which n is an integer between 1 and 6 inclusive and R₄ and R₅, which are identical or different, represent a hydrogen atom or a C₁-C₄ alkyl, C₁-C₄ hydroxyalkyl or C₁-C₆ acyl radical;
R₁ and R₂, which are identical or different, represent a hydrogen atom, a C₁-C₆ alkyl radical, a C₁-C₆ monohydroxyalkyl radical, a C₂-C₆ polyhydroxyalkyl radical, a C₁-C₆ monocarbamoylalkyl radical, a C₁-C₆ dialkylcarbamoyl radical, a C₁-C₆ aminoalkyl radical, a C₁-C₆ acyl radical, a C₂-C₆ carbalkoxy radical, a carbamoyl radical or a C₁-C₆ monoalkylcarbamoyl radical;
R₃ represents a hydrogen atom, a C₁-C₄ alkyl radical, a nitrile radical, an amide radical, a C₁-C₄ fluoroalkyl radical or a radical -COOR where R represents hydrogen or a C₁-C₄ alkyl radical, and the acid salts corresponding to the compounds of formula (I), in the presence of a oxidizing agent.

14. Process according to claim 13, characterized in that a dyeing composition for keratinous fibers, and in particular human hair, containing, in a medium suitable for dyeing, at least one oxidation dye precursor of ortho and/or para type and at least, as coupler, one 2-sulfurated metaphenylenediamine as defined in claim 13 is mixed, at the time of use, with an oxidizing solution in an amount sufficient to develop the coloring, the resulting composition having a pH varying between 3 and 11, and the mixture thus obtained is applied to keratinous fibers and in particular human hair.

15. Process according to claim 13, characterized in that, in a first stage, the oxidation dye precursor of ortho and/or para type or their mixture is applied to keratinous fibers, in a second stage, a composition containing the coupler of formula (I) is applied and the coloring is developed using an oxidizing agent, present in the said composition or else applied to the keratinous fibers in a third stage.

16. Oxidation dyeing process according to any one of claims 13 to 15, characterized in that exposure is allowed to take place for 10 to 40 minutes, preferably 15 to 30 minutes, the hair is rinsed, is washed with shampoo, is rinsed again and is dried.

17. 2-Sulfurated metaphenylenediamine of general formula: 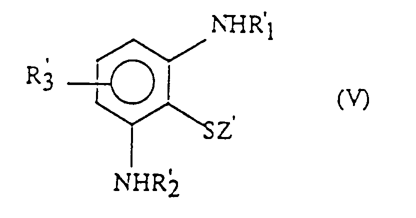 in which: Z' denotes a C₁-C₁₈ alkyl radical, an aralkyl radical in which the alkyl radical is C₁-C₆, a C₁-C₆ monohydroxyalkyl or C₂-C₆ polyhydroxyalkyl radical, an aryl radical or an aminoalkyl radical of formula: 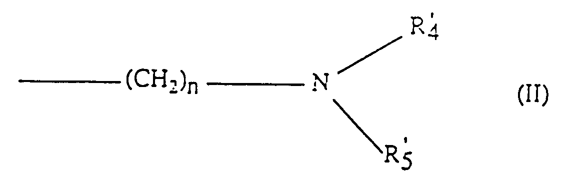 in which n is an integer between 1 and 6 inclusive; R'₄ and R'₅, which are identical or different, represent a hydrogen atom or a C₁-C₄ alkyl, C₁-C₄ hydroxyalkyl or C₁-C₆ acyl radical;
R'₁ and R'₂, which are identical or different, denote a hydrogen atom, a C₁-C₆ alkyl radical, a C₁-C₆ monohydroxyalkyl radical, a C₂-C₆ polyhydroxyalkyl radical, a C₁-C₆ monocarbamoylalkyl radical, a C₁-C₆ dialkylcarbamoyl radical, a C₁-C₆ aminoalkyl radical, a C₁-C₆ acyl radical, a C₂-C₆ carbalkoxy radical, a carbamoyl radical or a C₁-C₆ monoalkylcarbamoyl radical;
R'₃ represents a hydrogen atom or a C₁-C₄ alkyl, nitrile , amide , C₁-C₄ fluoroalkyl or -COOR radical where R represents a hydrogen atom or a C₁-C₄ alkyl radical;
and their acid salts
with the proviso that:
· when R'₁ and R'₂ and R'₃ represent a hydrogen atom, Z' is other than the alkyl radical or the aryl radical;
· when Z' represents the methyl radical and R'₃ represents a hydrogen atom, R'₁ and R'₂ cannot both represent the same C₁-C₆ acyl radical;
· when Z' represents the methyl radical and when R'₃ represents a hydrogen atom and when one of the two R'₁ and R'₂ radicals represents a hydrogen atom, the other R'₁ or R'₂ radical cannot represent a C₄ acyl radical;
· when R'₁ and R'₂ represent a hydrogen atom and R'₃ a trifluoromethyl radical in the 5 position, Z' cannot represent an alkyl-radical ;
· when R'₁ and R'₂ represent a hydrogen atom and R'₃ represents the methyl radical in the 4 position, Z' cannot represent a methyl radical.

18. 2-Sulfurated metaphenylenediamine according to claim 17, characterized in that it is chosen from 2-(β-acetylaminoethylthio)-1,3-diaminobenzene, 2-methylthio-5-methyl-1,3-diaminobenzene, 2-methylthio-5-amido-1,3-diaminobenzene and 2-methylthio-5-nitrilo-1,3-diaminobenzene .

19. Dyeing composition for keratinous fibers and in particular human hair characterized in that it contains, in a medium suitable for dyeing, at least one 2-sulfurated metaphenylenediamine of formula (V) as defined in one of claims 17 or 18.

## Patentansprüche

1. Zur Färbung keratinischer Fasern und insbesondere der menschlichen Haare vorgesehene Verwendung mindestens eines in Position 2 einen schwefelhaltigen Substituenten aufweisenden n-Phenylendiamins der allgemeinen Formel: 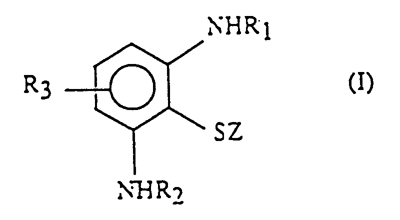 worin gilt:
Z stellt einen C₁₋₁₈-Alkylrest, einen Aralkylrest, worin der Alkylrest ein C₁₋₆-Alkylrest ist, einen Monohydroxy-C₁₋₆-alkyl- oder Polyhydroxy-C₂₋₆-alkyl-, Aryl- oder einen Aminoalkylrest der Formel dar: 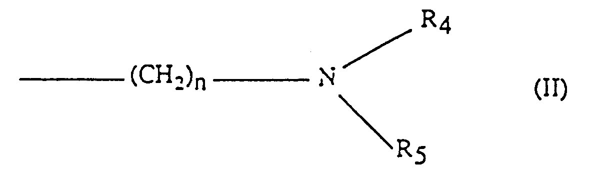 worin n eine ganze Zahl von 1 bis 6 ist; R₄ und R₅ stellen, gleich oder verschieden, ein Wasserstoffatom oder einen C₁₋₄-Alkyl-, Hydroxy-C₁₋₄-alkyl- oder einen C₁₋₆-Acylrest dar;
R₁ und R₂ stellen, gleich oder verschieden, ein Wasserstoffatom, einen C₁₋₆-Alkyl-, Monohydroxy-C₁₋₆-alkyl-, Polyhydroxy-C₂₋₆-alkyl-, Monocarbamyl-C₁₋₆-alkyl-, C₁₋₆-Dialkylcarbamyl-, Amino-C₁₋₆-alkyl-, C₁₋₆-Acyl-, C₂₋₆-Carbalkoxy-, Carbamyl- oder einen C₁₋₆-Monoalkylcarbamylrest dar;
R₃ stellt ein Wasserstoffatom, einen C₁₋₄-Alkyl-, Nitril-, Amid-, C₁₋₄-Fluoralkyl- oder einen -COOR-Rest dar, worin R ein Wasserstoffatom oder einen C₁₋₄-Alkylrest darstellt,
sowie die Verwendung von Säuresalzen, die den Verbindungen der Formel (I) entsprechen.

2. Verwendung gemäß Anspruch 1,
dadurch **gekennzeichnet**, daß
das in Position 2 einen schwefelhaltigen Substituenten aufweisende m-Phenylendiamin der Formel (I) in Gegenwart von Oxidationsfarbstoff-Vorstufenverbindungen vom o- und/oder p-Typ zur Anwendung gelangt.

3. Verwendung gemäß Anspruch 1 oder 2,
dadurch **gekennzeichnet**, daß
in der Verbindung der Formel (I) Z einen Methyl-, Ethyl-, Propyl-, Butyl-, Dodecyl-, Hexadecyl-, Benzyl-, Phenyl- oder einen Mono- oder Polyhydroxyalkylrest, ausgewählt aus: -CH₂-CH₂OH, -CH₂-CHOH-CH₂OH, -CH₂CHOH-CH₃,
oder einen Aminoalkylrest bedeutet, ausgewählt aus : CH₂-CH₂-NH₂, -CH₂-CH₂-NHCH₃, -CH₂-CH₂-N-CH₃-COCH₃, -CH₂-CH₂-NHCOCH₃;
und wenn die Gruppen R₁, R₂, R₄ und R₅ einen Acylrest bedeuten, dieser eine Formyl-, Acetyl- und Propionylgruppe darstellt, und R₃ die in Anspruch 1 definierte Bedeutung hat.

4. Verwendung gemäß jedem der Ansprüche 1 bis 3,
dadurch **gekennzeichnet**, daß
die Verbindungen der Formel (I) aus den folgenden Verbindungen ausgewählt sind:
- 2-β-Acetylaminoethylthio-1,3-diaminobenzol,
- 2-Methylthio-5-methyl-1,3-diaminobenzol,
- 2-Methylthio-5-amido-1,3-diaminobenzol,
- 2-Methylthio-5-nitrilo-1,3-diaminobenzol,
- 2-Methylthio-5-trifluormethyl-1,3-diaminobenzol,
- 2-Methylthio-1,3-diaminobenzol,
- 2-Methylthio-3-amino-1-carbamyl-t-butylbenzol,
- 2-Methylthio-1,3-di(carbamyl-t-butyl)benzol,
- 2-Methylthio-1,3-diacetylaminobenzol,
- 2-n-Propylthio-5-trifluormethyl-1,3-diaminobenzol,
- 2-Isopropylthio-5-trifluormethyl-1,3-diaminobenzol,
- 2-Methylthio-4-methyl-1,3-diaminobenzol,
- 2-Ethylthio-5-trifluormethyl-1,3-diaminobenzol,
- 2-Isobutylthio-5-trifluormethyl-1,3-diaminobenzol,
- 2-n-Butylthio-5-trifluormethyl-1,3-diaminobenzol,
und aus deren Säuresalzen.

5. Verwendung gemäß jedem der Ansprüche 1 bis 4,
dadurch **gekennzeichnet**, daß
die entsprechenden Säuresalze aus Hydrochloriden, Sulfaten oder Hydrobromiden ausgewählt sind.

6. Verwendung gemäß jedem der Ansprüche 2 bis 5,
dadurch **gekennzeichnet**, daß
die Oxidationsfarbstoff-Vorstufenverbindungen aus p-Phenylendiaminen, p-Aminophenolen, heterozyklischen Vorstufenverbindungen aus para-Derivaten des Pyridins oder Pyrimidins, o-Aminophenolen und aus Bisphenylalkylendiaminen ausgewählt sind.

7. Zusammensetzung zur Färbung keratinischer Fasern und insbesondere der menschlichen Haare, welche in einem zur Färbung geeigneten Milieu mindestens eine Oxidationsfarbstoff-Vorstufenverbindung vom o- oder p-Typ und mindestens, als Kuppler, ein in den Ansprüchen 1, 3 oder 4 definiertes m-Phenylendiamin enthält, das in Position 2 einen schwefelhaltigen Substituenten aufweist.

8. Färbezusammensetzung gemäß Anspruch 7,
dadurch **gekennzeichnet**, daß
sie Kuppler wie m-Diphenole, m-Aminophenole, m-Phenylendiamine, die sich von denen der Formel (I) unterscheiden, m-Acylaminophenole, m-Ureidophenole, m-Carbalkoxyaminophenole, α-Naphthol, Indolderivate und Kuppler mit einer aktiven Methylengruppe enthält.

9. Färbezusammensetzung gemäß jedem der Ansprüche 7 bis 8,
dadurch **gekennzeichnet**, daß
sie 0,05 bis 3,5 Gew.% des Gesamtgewichts der Zusammensetzung mindestens einer Verbindung der Formel (I) enthält.

10. Färbezusammensetzung gemäß jedem der Ansprüche 7 bis 9,
dadurch **gekennzeichnet**, daß
sie 0,3 bis 7 Gew.%, bezogen auf das Gesamtgewicht der Zusammensetzung, an Oxidationsfarbstoff-Vorstufenverbindungen vom o- und/oder p-Typ und an Kupplern enthält.

11. Färbezusammensetzung gemäß jedem der Ansprüche 7 bis 10,
dadurch **gekennzeichnet**, daß
sie kationische, anionische, nicht-ionische oder amphotere oberflächenaktive Mittel oder deren Mischungen in Konzentrationen von 0,5 bis 55 Gew.%, bezogen auf das Gesamtgewicht der Zusammensetzung, organische Lösungsmittel in Konzentrationen von 1 bis 40 Gew.%, bezogen auf das Gesamtgewicht der Zusammensetzung, Direktfarbstoffe, Verdickungsmittel in Konzentrationen von 0,1 bis 5 Gew.%, bezogen auf das Gesamtgewicht der Zusammensetzung, und antioxidierende Mittel in Mengenanteilen von 0,05 bis 1,5 Gew.%, bezogen auf das Gesamtgewicht der Zusammensetzung, enthält.

12. Färbezusammensetzung gemäß jedem der Ansprüche 7 bis 11,
dadurch **gekennzeichnet**, daß
sie in Form einer Flüssigkeit, Creme oder eines Gels oder in jeder weiteren geeigneten Form vorliegt oder als Aerosol-Fläschchen in Gegenwart eines Treibmittels zubereitet sein kann und Schäume bildet.

13. Verfahren zur Oxidationsfärbung keratinischer Fasern und insbesondere der menschlichen Haare,
dadurch **gekennzeichnet**, daß
man auf die keratinischen Fasern mindestens eine Oxidationsfarbstoff-Vorstufenverbindung vom o- und/oder p-Typ und ein in Position 2 einen schwefelhaltigen Substituenten aufweisendes m-Phenylendiamin der allgemeinen Formel: 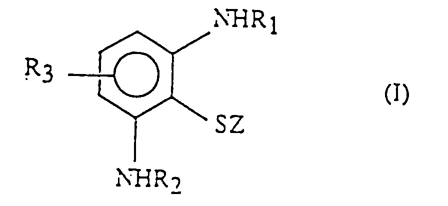 worin gilt:
Z stellt einen C₁₋₁₈-Alkylrest, einen Aralkylrest, worin der Alkylrest ein C₁₋₆-Alkylrest ist, einen Monohydroxy-C₁₋₆-alkyl- oder Polyhydroxy-C₂₋₆-alkyl-, Aryl- oder einen Aminoalkylrest der Formel dar: 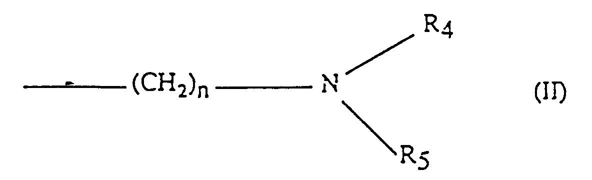 worin n eine ganze Zahl von 1 bis 6 ist; R₄ und R₅ stellen, gleich oder verschieden, ein Wasserstoffatom oder einen C₁₋₄-Alkyl-, Hydroxy-C₁₋₄-alkyl- oder einen C₁₋₆-Acylrest dar;
R₁ und R₂ stellen, gleich oder verschieden, ein Wasserstoffatom, einen C₁₋₆-Alkyl-, Monohydroxy-C₁₋₆-alkyl-, Polyhydroxy-C₂₋₆-alkyl-, Monocarbamyl-C₁₋₆-alkyl-, C₁₋₆-Dialkylcarbamyl-, Amino-C₁₋₆-alkyl-, C₁₋₆-Acyl-, C₂₋₆-Carbalkoxy-, Carbamyl- oder einen C₁₋₆-Monoalkylcarbamylrest dar;
R₃ stellt ein Wasserstoffatom, einen C₁₋₄-Alkyl-, Nitril-, Amid-, C₁₋₄-Fluoralkyl- oder einen -COOR-Rest dar, worin R ein Wasserstoffatom oder einen C₁₋₄-Alkylrest darstellt;
sowie die Säuresalzen, die den Verbindungen der Formel (I) entsprechen, in Gegenwart eines oxidierenden Mittels aufbringt.

14. Verfahren gemäß Anspruch 13,
dadurch **gekennzeichnet**, daß
man, zum Zeitpunkt der Anwendung, eine Färbezusammensetzung für keratinische Fasern und insbesondere für die menschlichen Haare, die, in einem zur Färbung geeigneten Milieu, mindestens eine Oxidationsfarbstoff-Vorstufenverbindung vom o- und/oder p-Typ und mindestens, als Kuppler, ein in Anspruch 13 definiertes m-Phenylendiamin, das in Position 2 einen schwefelhaltigen Substituenten aufweist, mit einer oxidierenden Lösung in einer zur Entwicklung der Färbung ausreichenden Menge vermischt, wobei die sich ergebende Zusammensetzung einen pH-Wert von 3 bis 11 aufweist, und daß man die so erhaltene Mischung auf die keratinischen Fasern und insbesondere die menschlichen Hare aufbringt.

15. Verfahren gemäß Anspruch 13,
dadurch **gekennzeichnet**, daß
man in einer ersten Stufe die Oxidationsfarbstoff-Vorstufenverbindung vom o- und/oder p-Typ oder deren Mischung auf die keratinischen Fasern und in einer zweiten Stufe eine Zusammensetzung aufbringt, die den Kuppler der Formel (I) enthält, und daß man die Färbung mit einem oxidierenden Mittel entwickelt, das in der genannten Zusammensetzung vorhanden ist oder auch auf die keratinischen Fasern in einer dritten Stufe aufgebracht wird.

16. Verfahren zur Oxidationsfärbung gemäß jedem der Ansprüche 13 bis 15,
dadurch **gekennzeichnet**, daß
man die jeweiligen Zusammensetzungen 10 bis 40 und vorzugsweise 15 bis 30 Minuten lang verweilen läßt, dann die Haare spült, sie unter Schamponieren wäscht, erneut spült und trocknet.

17. In Position 2 einen schwefelhaltigen Substituenten aufweisendes m-Phenylendiamin der allgemeinen Formel: 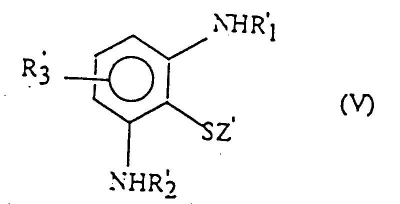 worin gilt:
Z' stellt einen C₁₋₁₈-Alkyl-, einen Aralkylrest mit einem C₁₋₆-Alkylrest, einen Monohydroxy-C₁₋₆-alkyl- oder Polyhydroxy-C₂₋₆-alkylrest, einen Aryl- oder einen Aminoalkylrest der Formel dar: 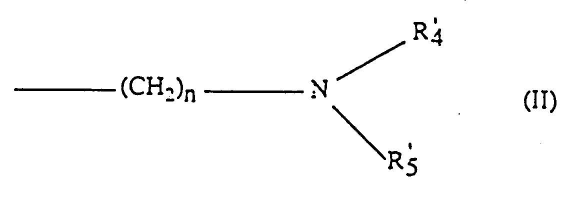 worin n eine ganze Zahl von 1 bis 6 ist und R'₄ und R'₅, gleich oder verschieden, ein Wasserstoffatom oder einen C₁₋₄-Alkyl-, Hydroxy-C₁₋₄-alkyl- oder einen C₁₋₆-Acylrest darstellen;
R'₁ und R'₂ stellen, gleich oder verschieden, ein Wasserstoffatom, einen C₁₋₆-Alkyl-, Monohydroxy-C₁₋₆-Alkyl-, Polyhydroxy-C₂₋₆-alkyl-, Monocarbamyl- C₁₋₆-alkyl-, C₁₋₆-Dialkylcarbamyl-, C₁₋₆-Aminoalkyl-, C₁₋₆-Acyl-, C₂₋₆-Carbalkoxy-, Carbamyl- oder einen C₁₋₆-Monoalkylcarbamylrest dar;
R'₃ stellt ein Wasserstoffatom, einen C₁₋₄-Alkyl-, Nitril-, Amid-, C₁₋₄-Fluoralkyl- oder einen -COOR-Rest dar, worin R ein Wasserstoffatom oder einen C₁₋₄-Alkylrest darstellt;
sowie deren Säuresalze,
mit der Maßgabe, daß:
- wenn R'₁, R'₂ und R'₃ ein Waserstoffatom darstellen, Z' sich von einem Alkyl- oder Arylrest unterscheidet;
- wenn Z' einen Methylrest und R'₃ Wasserstoff darstellen, R'₁ und R'₂ nicht gleichzeitig denselben C₁₋₆-Acylrest darstellen;
- wenn Z' einen Methylrest und R'₃ Wasserstoff und einer der beiden Reste R'₁ und R'₂ ein Wasserstoffatom darstellen, der andere Rest R'₁ oder R'₂ keinen C₄-Acylrest darstellen;
- wenn R'₁ und R'₂ Wasserstoff und R'₃ einen Trifluormethylrest in Position 5 darstellen, Z' keinen Alkylrest darstellt;
- und wenn R'₁ und R'₂ Wasserstoff und R'₃ einen Methylrest in Position 4 darstellen, Z' keinen Methylrest bedeutet.

18. In Position 2 einen schwefelhaltigen Substituenten aufweisendes m-Phenylendiamin gemäß Anspruch 17,
dadurch **gekennzeichnet**, daß
es aus 2-β-Acetylaminoethylthio-1,3-diaminobenzol, 2-Methylthio-5-methyl-1,3-diaminobenzol, 2-Methylthio-5-amido-1,3-diaminobenzol und aus 2-Methylthio-5-nitrilo-1,3-diaminobenzol ausgewählt ist.

19. Färbezusammensetzung für keratinische Fasern und insbesondere die menschlichen Haare,
dadurch **gekennzeichnet**, daß
sie, in einem zur Färbung geeigneten Milieu, mindestens ein in einem der Ansprüche 17 oder 18 definiertes m-Phenylendiamin der Formel (V) enthält, das in Position 2 einen schwefelhaltigen Substituenten aufweist.
